(19) Europäisches Patentamt
European Patent Office
Office européen des brevets

(11) Publication number: **0 435 229 A1**

(12) # EUROPEAN PATENT APPLICATION

(21) Application number: **90125293.2**

(22) Date of filing: **21.12.90**

(51) Int. Cl.5: **C07K 15/00**, C12N 15/40, C12Q 1/70, A61K 39/29

The applicant has subsequently filed a sequence listing and declared, that it includes no new matter.

(30) Priority: **27.12.89 JP 344876/89**
**09.02.90 JP 28534/90**

(43) Date of publication of application:
**03.07.91 Bulletin 91/27**

(84) Designated Contracting States:
**AT BE CH DE DK ES FR GB GR IT LI LU NL SE**

(71) Applicant: **SANWA KAGAKU KENKYUSHO CO., LTD.**
**No. 35, Higashi-sotobori-cho, Higashi-ku**
**Nagoya, Aichi-ken(JP)**

(72) Inventor: **Kobayashi, Kenichi**
**2-10-36, Nagadohe**
**Kanazawa-shi, Ishikawa-ken(JP)**
Inventor: **Murakami, Seishi**
**12-13 Wa, Okuwa-machi**
**Kanazawa-shi, Ishikawa-ken(JP)**
Inventor: **Kaneko, Shuichi**
**4-13-21, Izumino-machi**
**Kanazawa-shi, Ishikawa-ken(JP)**
Inventor: **Sawai, Kiichi**
**35, Higashi-sotobori-cho, Higashi-ku**
**Nagoya, Aichi-ken(JP)**

(74) Representative: **Wächtershäuser, Günter, Dr.**
**Tal 29**
**W-8000 München 2(DE)**

(54) **DNA of non-A, non-B hepatitis virus gene, its clone and use thereof.**

(57) A family of DNAs derived from non-A, non-B hepatitis (NANBH) virus gene is provided. Each of the DNAs has about 210 to 240 deoxyribonucleotides and is prepared by obtaining and purifying RNA fraction derived from a plasma sample of an NANBH patient, separating mRNA from the RNA fraction, and synthesizing into the DNA with use of the mRNA as a template. A diagnosis of NANBH is done through a polymerase chain reaction method which uses a DNA synthesized by starting from RNA derived from a serum sample of a person to be diagnosed and a set of primers, each of which primers is a DNA fragment constituting a part of the DNA having about 210 to 240 deoxyribonucleotides, and through a judgement of whether a predetermined band can be detected or not in a pattern of electrophoresis.

EP 0 435 229 A1

# DNA OF NON-A, NON-B HEPATITIS VIRUS GENE, ITS CLONE AND USE THEREOF

The invention relates to a DNA of non-A, non-B hepatitis (hereinafter referred merely to "NANBH") virus gene, its clone and use thereof for diagnosing the NANBH.

At the present time, the NANBH occupies 90 to 95% among a family of blood transfusional hepatitises, and a positive remedy thereof has not yet been established. Therefore, there is a strong demand for developments of its diagnosing method, effective medicine, agent for treating a transfusional blood to inactivate or kill possible NANBH virus therein, preventive vaccine and others.

In recent years, a search on NANBH virus antigens great progresses and several reports have been issued. Namely, in Jap. Pat. Nos. Sho 62 - 249999(A) and Sho 63 - 91328(A), there is given a statement that a single-stranded RNA having about 10000 deoxyribonucleotides may concern to NANBH virus gene. While, Michael HOUGTON et al in Chiron Corporation located at California, U.S.A. have made a cDNA derived from a liver tissue sample of a chimpanzee infected with NANBH into its clone, and expressed the same in Escherichia coli with λ gt11 vector transformed by the cloned DNA, to find that a product thereof reacts with a serum sample derived from an NANBH patient. They have finally confirmed that the product is a single-stranded RNA having about 9000 deoxyribonucleotides, which is similar to that for filavi virus, named the same as "HCV", and reported a part of nucleotide sequence for the cloned DNA (about 7000 deoxyribonucleotides among said about 9000 deoxyribonucleotides) [EP 0318216(A1)].

Further, Chiron Corporation has developed and marketed a diagnosis kit which comprises a yeast transformed by a part of the HCV gene, said yeast producing a polypeptide which acts as an antigen reacting with an antibody in a serum sample derived from a patient infected with NANBH, and they said that the kit is employed to judge whether a patient with NANBH was infected with HCV or not, and that the judgement can be passed with about 70% in immunological probability.

Said method diagnoses whether the NANBH patient has been infected with HCV or not, and therefore, it can not be judged whether the NANBH patient is, at the present time, infected with HCV. Since said HCV gene is a virus gene derived from a plasma sample of a chimpanzee being infected with a blood let from an american individual with NANBH and host is the chimpanzee, further, such a possibility can not be negated that the HCV gene in question is different from a nature type human NANBH virus gene, due to a variation in body of the host. In fact, an immunological search using the diagnosis kit marketed from Chiron Corporation shows that it can not always be obtained a satisfiable result.

Further, a recent newspaper has introduced such a report made by Terukatsu ARIMA et al of Okayama University, Japan that a fragment of NANBH virus gene, which corresponds to about a half of entire gene has been separated and elucidated, but it has not yet been confirmed as an actual NANBH virus gene.

A basic object of the invention is to provide a DNA of at least a part of human NANBH virus gene, which has no variation from nature one or less in variation.

An additional object of the invention is to provide a cloned DNA of at least a part of human NANBH virus gene.

Another additional object of the invention is to provide a method of diagnosing NANBH, which uses the cloned DNA.

In view of the basic object of the invention, the inventors have energetically studied and investigated on a cloning of NANBH virus gene, which starts from a blood, and more particularly plasma derived from individuals infected with NANBH and is carried out without through another animal.

As a result, the inventors have found a single-stranded DNA having about 5000 deoxyribonucleotides, which has been estimated to have a nucleotide sequence encoding at least a part of amino acid sequence of the nature human NANBH virus gene, and a double-stranded DNA consisting of said single-stranded DNA and its complementary single-stranded DNA.

Each of single-stranded DNAs according to the invention and constituting a family of NANBH virus genes is a part of said single-stranded DNA with about 5000 deoxyribonucleotides, and has about 210 - 240 deoxyribonucleotides.

The single-stranded DNA according to the invention has a nucleotide sequence selected from the group consisting of

```
          10        20        30        40        50        60
A  CAGGAGTTTGATGAGATGGAGGAGTGCGCCTCACACCTTCCTTACATCGAACAAGGAATG
B  CGGGAGTTCGATGAGATGGAGGAGTGCGCTTCACACCTCCCTTACATCGAACAGGGAATG
C  CAAGAGTTCGACGAAATGGAAGAGTGCGCCTCACACCTTCCTTACATCGAACAGGGAATG
D  CCGGCGTTCGATGAAATGGAAGAGTGTGCCTCACACCTCCCTTACATCGAACAAGGAATG
E  CAGGAGTTTGATGAGATGGAGGAGTGCGCCTCACACCTTCCTTACATCGAACAAGGAATG
F  CAGGAGTTTGATGAGATGGAGGAGTGCGCCTCACACCTTCCTTACATCGAACAAGGAATG
G  CAGGAGTTTGATGAGATGGAGGAGTGCGCCTCACACCTTCCTTACATCGAACAAGGAATG

          70        80        90       100       110       120
A  CAGCTCGCCGAGCAATTCAAGCAGAAGGCGCTCGGGCTGCTGCAAACAGCCTCCAAGCAA
B  CAGCTCGCCGAGCAATTCAAGCAGAAGGCGCTCGGGCTGTTGCAGACAGCCACCAAGCAA
C  CAGCTCGCCGAGCAATTCAAGCAGAAAGCGCTCGGTTTGCTGCAGACAGCTACCAAGCAA
D  CGGCTCGCCGAACAATTCAAGCAGAAGGCGCTCGGGTTGCTGCAAACGGCCACCAAGCAA
E  CAGCTCGCCGAGCAATTCAAGCAGAAGGCGCTCGGGCTGCTGCAAACGGCCTCCAAGCAA
F  CAGCTCGCCGAGCAATTCAAGCAGAAGGCGCTCGGGCTGCTGCAAACAACCTCCAAGCAA
G  CAGCTCGCCGAGCAATTCAAGCAGAAGGCGCTCGGGCTGCTGCAGACAACCTCCAAGCAA

         130       140       150       160       170       180
A  GCGGAGGCTGCTGCTCCCGTGGTAGAGTCCAAGTGGCAAGCCCTTGAGGCCTTCTGGGCG
B  GCGGAGGCTGCAGCTCCCGTGGTCGAGTCTAAATGGCGGGCTCTTGAGACCTTCTGGGCG
C  GCGGAGGCTGCAGCCCCCGTGGTGGAGTCCAAGTGGCGGGCCCTTGAGACTTTCTGGGCG
D  GCGGAGGCTGCCGCTCCCGTGGTGGAGTCCAAGTGGCGTACCCTTGAGGCCTTCTGGGCG
E  GCGGAGGCTGCTGCTCCCGTGGTAGAGTCCAAGTGGCAAGCCCTTGAGGCCTTCTGGGCG
F  GCGGAGGCTGCTGCTCCTGTGGTAGAGTCCAAGTGGCAAGCCCTTGAGGCCTTCTGGGCG
G  GCGGAGGCTGCTGCTCCCGTGGTAGAGTCCAAGTGGCAAGCCCTTGAGGCCTTCTGGGCG

         190       200       210       220       230       240
A  AAGCACATGTGGAACTTCATCAGCGGGATACAGTATCTAGCAGGCTTGTCCACCCTGCCT
B  AAGCACATGTGGAATTTCATCAGCGGGATACAGTACCTAGCAGGCTTGTCCACTCTGCCT
C  AAGCACATGTGGAATTTCATCAGCGGGATACAATACTTAGCAGGCTTATCCACTTTGCCT
D  AAGCACATGTGGAATTTCATCAGCGGGATACAATACCTAGCAGGCTTGTCCACTCTGCCT
E  AAGCACATGTGGAATCTCATCAGCGGGATACAGTATCTAGCAGGCTTGTCCACCCTGCCT
F  AAGCACATGTGGAATCTCATCAGCGGGAT
G  AAGCACATGTGGAATCTCATCAGCGGGAT
```

wherein each of symbols of A, C, G and T is a deoxyribonucleotide having adenine, cytosine, guanine or thymine base.

In the nucleotide sequences, there are differences of deoxyribonucleotides only in 14 positions, namely at 2nd, 3rd, 5th, 108th, 109th, 111th, 112th, 158th, 159th, 160th, 162th, 169th, 171st and 196th. It may be estimated that the difference has been appeared due to differences in plasma samples derived from

different individuals who were definitely diagnosed as patient infected with NANBH and used for the cloning, or a possible variation or denaturation of nature human NANBH virus gene during various operations for the cloning. Even if the differences have been caused by the variation, a homology in said nucleotide sequences reaches about 95% or more and far excellent than that of about 70% homology as reported by Chiron Corporation.

Amino acid sequences translated from said nucleotide sequences are as follows.

| | 5 10 15 20 |
|---|---|
| A | GlnGluPheAspGluMetGluGluCysAlaSerHisLeuProTyrIleGluGlnGlyMet |
| B | ArgGluPheAspGluMetGluGluCysAlaSerHisLeuProTyrIleGluGlnGlyMet |
| C | GlnGluPheAspGluMetGluGluCysAlaSerHisLeuProTyrIleGluGlnGlyMet |
| D | ProAlaPheAspGluMetGluGluCysAlaSerHisLeuProTyrIleGluGlnGlyMet |
| E | GlnGluPheAspGluMetGluGluCysAlaSerHisLeuProTyrIleGluGlnGlyMet |
| F | GlnGluPheAspGluMetGluGluCysAlaSerHisLeuProTyrIleGluGlnGlyMet |
| G | GlnGluPheAspGluMetGluGluCysAlaSerHisLeuProTyrIleGluGlnGlyMet |

| | 25 30 35 40 |
|---|---|
| A | GlnLeuAlaGluGlnPheLysGlnLysAlaLeuGlyLeuLeuGlnThrAlaSerLysGln |
| B | GlnLeuAlaGluGlnPheLysGlnLysAlaLeuGlyLeuLeuGlnThrAlaThrLysGln |
| C | GlnLeuAlaGluGlnPheLysGlnLysAlaLeuGlyLeuLeuGlnThrAlaThrLysGln |
| D | GlnLeuAlaGluGlnPheLysGlnLysAlaLeuGlyLeuLeuGlnThrAlaThrLysGln |
| E | GlnLeuAlaGluGlnPheLysGlnLysAlaLeuGlyLeuLeuGlnThrAlaSerLysGln |
| F | GlnLeuAlaGluGlnPheLysGlnLysAlaLeuGlyLeuLeuGlnThrThrSerLysGln |
| G | GlnLeuAlaGluGlnPheLysGlnLysAlaLeuGlyLeuLeuGlnThrThrSerLysGln |

| | 45 50 55 60 |
|---|---|
| A | AlaGluAlaAlaAlaProValValGluSerLysTrpGlnAlaLeuGluAlaPheTrpAla |
| B | AlaGluAlaAlaAlaProValValGluSerLysTrpArgAlaLeuGluThrPheTrpAla |
| C | AlaGluAlaAlaAlaProValValGluSerLysTrpArgAlaLeuGluThrPheTrpAla |
| D | AlaGluAlaAlaAlaProValValGluSerLysTrpArgThrLeuGluAlaPheTrpAla |
| E | AlaGluAlaAlaAlaProValValGluSerLysTrpGlnAlaLeuGluAlaPheTrpAla |
| F | AlaGluAlaAlaAlaProValValGluSerLysTrpGlnAlaLeuGluAlaPheTrpAla |
| G | AlaGluAlaAlaAlaProValValGluSerLysTrpGlnAlaLeuGluAlaPheTrpAla |

| | 65 | 70 | 75 | 80 |
|---|---|---|---|---|
| A | LysHisMetTrpAsnPheIleSerGlyIleGlnTyrLeuAlaGlyLeuSerThrLeuPro | | | |
| B | LysHisMetTrpAsnPheIleSerGlyIleGlnTyrLeuAlaGlyLeuSerThrLeuPro | | | |
| C | LysHisMetTrpAsnPheIleSerGlyIleGlnTyrLeuAlaGlyLeuSerThrLeuPro | | | |
| D | LysHisMetTrpAsnPheIleSerGlyIleGlnTyrLeuAlaGlyLeuSerThrLeuPro | | | |
| E | LysHisMetTrpAsnLeuIleSerGlyIleGlnTyrLeuAlaGlyLeuSerThrLeuPro | | | |
| F | LysHisMetTrpAsnLeuIleSerGly | | | |
| G | LysHisMetTrpAsnLeuIleSerGly | | | |

Each of the double-stranded DNAs containing the single-stranded DNA with about 210 or 240 deoxyribonucleotides can be prepared by extracting RNA from a granular fraction of a plasma sample derived from a patient infected with NANBH, purifying the RNA, and carrying out two-stage strand synthesis with use of conventional techniques known in the art and the purified RNA as a template.

The resulting double-stranded DNA can be inserted with a conventional techniques into an expression vector such as a phage or plasmid vector to obtain a cloned DNA. For carrying out the cloning, it is convenient to employ an in vitro packaging method, when the phage is selected as the vector. Microorganisms such as Escherichia coli and yeast, or animal cells can be transformed with the recombinant vector to produce an antigen due to NANBH virus gene through a cultivation of the microorganism or animal cell. If a toxicity of the antigenous material can be weaken through conventional techniques such as a subcluturing method, a vaccine to NANBH will be obtained. Further, the vaccine may be administered to an experimental animal to produce an antibody material which can be taken from the animal to prepare a remedy to NANBH and an agent for treating a transfusional blood. The antibody material can be made into monoclonal or polyclonal one to supply the remedy in a stable manner and large amount. If a series of dilutions shall be prepared with use of the antibody material, a stage of advance or recovery of the hepatitis and cancer or tumor due to NANBH virus can be judged with a high probability.

According to the invention, a diagnosis of NANBH can be done by carrying out a polymerase chain reaction (PCR) method (method for detecting a virus marker, which has recently been developed by Cetus Corporation, U.S.A.), in accordance with the description given by KANEKO et al ["Journal of Clinical Microbiology", Vol. 27., pages 1930 - 1933 (1989)] with use of primers which are fragments of the cloned DNA as well as a RNA derived from a serum sample of an individual to be diagnosed, since if the individual is the carrier of NANBH virus, a result of electrophoresis shows a predetermined band. but no such band can be detected, when an RNA employed has been prepared from a serum sample of a normal individual not infected with NANBH.

The invention will now be further explained in more detail with reference to Examples which shall refer to drawings, wherein

Fig. 1 shows 7 single-stranded nucleotide sequences of cDNA clones derived from plasma samples of different individuals who had definitely been diagnosed as patients with NANBH;

Fig. 2 shows amino acid sequences translated from the nucleotide sequences given in Fig. 1;

Fig. 3 is patterns showing results of electrophoresis after polymerase chain reaction which uses serum samples of 4 different individuals who had definitely been diagnosed as patients infected with NANBH and primers which are fragments of single-stranded cDNA clone shown as A in Fig. 1; and

Fig. 4 is patterns similar to those in Fig. 3, but with use of serum samples derived from other 2 patients and primers which are fragments of single-stranded cDNA clone shown as F in Fig. 1.

Example 1

a) Separation and purification of RNA containing mRNA of NANBH virus

In each of 7 plasma samples derived from 7 different individuals who had definitely been diagnosed as patients infected with NANBH, 20% polyethylene glycol (PEG) solution in TEN (TEN buffer) was added to stir and left to stand for 30min. Then, the solution was high speed centrifuged to obtain a precipitational fraction.

The fraction was solubilized by adding TEN in an amount of 5 folds and high speed centrifuged to remove a none-soluble fraction.

The resulting solution was put on a cushion of 15% and 60% sucrose solution in TEN and supercentrifuged at 25000rpm for 12 hours, with use of SW 28 rotor to obtain a granular fraction. After repeated the operation, the resulting granular fraction was put on another cushion of 15% sucrose solution in TEN and supercentrifuged at 25000rpm for 12 hours, with use of SW 28 rotor to obtain a granular fraction which was dissolved in TEN.

GIT was added to the solution and the resulting mixture was put on a cushion of CsCl in TEN (density : 1.7) and supercentrifuged at 35000rpm for 12 hours, with use of SW 28 rotor to obtain a precipitational fraction which shall be used as "RNA fraction".

A double stage extraction of RNA were applied to the RNA fraction, with use of firstly a mixture of chloroform and phenol, and secondary chloroform. In a separated aqueous solution, glycogen as a carrier was gradually added (final concentration : 20$\mu$ g/ml) and then ethanol was added to cause a precipitation. The resulting solution with the precipitation was centrifuged to obtain a precipitational fraction as a desirous purified RNA.

b) Separation of mRNA from purified RNA and synthesis of DNA

The first stage strand synthesis was carried out by using oligo(dT) as a primer (random hexamers may also be employed for this purpose) to the purified RNA obtained in Item (a), and incuvating the same at 42°C for 45min., in the presence of a reverse transcriptase (20U). This operation provides a double-stranded product consisting of the RNA as the template and its complementary single-stranded DNA (cDNA).

In the next place, the second strand synthesis was carried out by adding to the resulting reaction mixture an RNase H (0.8U) and a DNA polymerase (23U) and incuvating the resulting mixture through three stages, namely at 12°C for 60min., 22°C for 60min., and 70°C for 10min. Through this operation, the single-stranded cDNA is separated from the product in the first strand synthesis and a desired double-stranded DNA is synthesized, which the last mentioned DNA consists of said separated single-stranded cDNA and its complementary single-stranded DNA.

Then, $T_4$ DNA polymerase (2.0U) was added to the resulting reaction mixture to incuvate at 37°C for 10min., so that each end of the DNA fragment was blunted.

To the DNA fragment extracted with phenol and chloroform, EcoRI linker was ligated at both ends with use of $T_4$ ligase, digested with EcoRI, and then purified to obtain a desired double-stranded DNA fragment.

This double-stranded DNA fragment can be separated into constitutional single-stranded DNAs, in a conventional manner.

c) Insertion of DNA into vector

A phage vector of λ gt11 was selected and cleaved with EcoRI. To the cleaved vector, the DNA fragment obtained in Item (b) and with EcoRI recognition site at both ends was ligated. The resulting recombinant vector was screened by carrying out in vitro packaging method to isolate and obtain a phage vector clone with said DNA insert.

d) Identification of DNA fragment, as clone

Each of the DNA fragments derived from 7 patients was separated into its constitutional single-stranded DNAs and cleaved to obtain several short fragments.

PCR method was applied with use of the resulting fragments as primers and RNA fraction derived from a serum sample of another patient infected with NANBH to detect a predetermined band, as a result of electrophoresis. This means that each of the larger DNA fragments is a clone having a part of NANBH virus gene, since the band can not be detected, when another RNA derived from a serum sample of a normal individual who is not infected with NANBH.

e) Determination of nucleotide sequence and amino acid sequence

A nucleotide sequence of each DNA clone obtained in Item (c) and confirmed in Item (d) was checked in accordance with the dideoxy method. A part of each nucleotide sequence, which is subject matter of the invention, is shown in Fig. 1.

As apparently seen from the Figure, a homology of the constitutional deoxyribonucleotides in the sequences reaches to about 95% or more.

In Fig. 2, there are shown amino acid sequences translated from the nucleotide sequences given in Fig. 1. As apparently seen from this Figure, there are deferences in amino acids only in 8 positions, namely at 1st, 2nd, 37th, 38th, 53rd, 54th, 57th and 66th, and a homology thereof reaches about 90% or more. More particularly, the last 3 amino acid sequences (E, F and G) are common, with one another.

Example 2

Entire RNA was extracted from 4 serum samples derived from different 4 patients definitely diagnosed as infected with NANBH. This extraction can be done in accordance with the method using guanidine thiocyanate and disclosed by Zirgwin in "Biochemistry", Vol. 18, pages 5294 - 5299 (1979) or using a vanadyl complex.

Each of the resulting RNA and primers of cDNA fragments corresponding to nucleotide sequences of 22nd to 57th deoxyribonucleotides in A of Fig. 1 and 174th to 209th deoxyribonucleotides, are incuvated at 42° C for 1 hour, in the presence of a transcriptase, to synthesize a complementary DNA fragment. The reaction mixture was subjected to a first polymerase chain reaction to amplify the DNA. This reaction can be made in accordance with the disclosure given by KANEKO et al in "Journal of Clinical Microbiology", Vol. 27, pages 1930 - 1933 (1989). A second polymerase chain reaction was carried out with use of other primers of cDNA fragments corresponding to nucleotide sequences of 37th to 57th deoxyribonucleotides in A of Fig. 1 and 174th to 194th deoxyribonucleotides to further amplify the DNA. Then, an electrophoresis was carried out to obtain patterns given in lanes 2 to 5 of Fig. 3 (Lane 1 shows patterns, when size markers employed in lieu of the cDNA). In the lanes 2 to 5, there is a band of about 100 base pairs, which shows that the person furnished the serum is infected with NANBH virus, since if a serum derived from a normal individual not infected with NANBH, is employed as the raw material for extracting RNA therein, no band of about 100 base pairs can be detected.

A second test was carried out with use of each 4 RNA samples derived from said 4 NANBH patients. In this case, first and second polymerase chain reactions were carried out with use of primers of cDNA fragments corresponding to nucleotide sequences of 1st to 36th deoxyribonucleotides and 127th to 162nd deoxyribonucleotides as well as 16th to 36th deoxyribonucleotides and 142nd to 162nd deoxyribonucleotides, respectively. A band of about 100 base pairs can also be detected in each case as shown in lanes 7 - 10 of Fig. 3. Please note that lane 6 of Fig. 3 shows patterns on size markers.

In Fig. 3, the patterns in lanes 2 and 7, 3 and 8 as well as 4 and 9 shows that when the serum of same NANBH patient is employed for extracting RNA therefrom.

Example 3

Entire RNA was extracted from serum samples derived from 2 patients definitely diagnosed as infected with NANBH.

The resulting RNA and primers of cDNA fragments corresponding to nucleotide sequences of 22nd to 57th deoxyribonucleotides in F of Fig. 1 and 174th to 209th deoxyribonucleotides, are incuvated at 42° C for 1 hour, in the presence of a transcriptase, to synthesize a complementary DNA. The reaction mixture was subjected to a first polymerase chain reaction to amplify the DNA. A second polymerase chain reaction was carried out with use of other primers of cDNA fragments corresponding to nucleotide sequences of 37th to 57th deoxyribonucleotides in F of Fig. 1 and 174th to 194th deoxyribonucleotides to further amplify the DNA. Then, an electrophoresis was carried out to obtain pattern given in lanes 2 and 3 of Fig. 4 (Lane 1 shows patterns, when size markers employed in lieu of the cDNA). In the lanes 2 and 3, there is a band of about 100 base pairs, which shows that the person furnished the serum is infected with NANBH virus, since if a serum derived from a normal individual not infected with NANBH, is employed as the raw material for extracting RNA therein, no band of about 100 base pairs can be detected.

A second test was carried out with use of each 2 RNA samples derived from said 2 NANBH patients. In this case, first and second polymerase chain reactions were carried out with use of primers of cDNA fragments corresponding to nucleotide sequences of 1st to 36th deoxyribonucleotides and 127th to 162nd deoxyribonucleotides as well as 16th to 36th deoxyribonucleotides and 142nd to 162nd deoxyribonucleotides, respectively. A band of about 100 base pairs can also be detected in both cases as shown in lanes 5 and 6 of Fig. 4. Please note that lane 4 of Fig. 4 shows patterns on size markers.

SEQ ID NO : 1
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
           10        20        30        40        50        60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
                5                  10                  15                  20

           70        80        90       100       110       120
CAGCTCGCCGAGCAATTCAAGCAGAAGGCGCTCGGGCTGCTGCAAACAGCCTCCAAGCAA
GlnLeuAlaGluGlnPheLysGlnLysAlaLeuGlyLeuLeuGlnThrAlaSerLysGln
               25                  30                  35                  40

          130       140       150       160       170       180
GCGGAGGCTGCTGCTCCCGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
               45                  50                  55                  60

          190       200       210       220       230       240
AAGCACATGTGGAACTTCATCAGCGGGATACAGTATCTAGCAGGCTTGTCCACCCTGCCT
LysHisMetTrpAsnPheIleSerGlyIleGlnTyrLeuAlaGlyLeuSerThrLeuPro
               65                  70                  75                  80
```

SEQ ID NO : 2
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
           10         20         30         40         50         60
CGGGAGTTCGATGAGATGGAGGAGTGCGCTTCACACCTCCCTTACATCGAACAGGGAATG
ArgGluPheAspGluMetGluGluCysAlaSerHisLeuProTyrIleGluGlnGlyMet
                5                  10                  15                  20

           70         80         90        100        110        120
CAGCTCGCCGAGCAATTCAAGCAGAAGGCGCTCGGGCTGTTGCAGACAGCCACCAAGCAA
GlnLeuAlaGluGlnPheLysGlnLysAlaLeuGlyLeuLeuGlnThrAlaThrLysGln
               25                  30                  35                  40

          130        140        150        160        170        180
GCGGAGGCTGCAGCTCCCGTGGTCGAGTCTAAATGGCGGGCTCTTGAGACCTTCTGGGCG
AlaGluAlaAlaAlaProValValGluSerLysTrpArgAlaLeuGluThrPheTrpAla
               45                  50                  55                  60

          190        200        210        220        230        240
AAGCACATGTGGAATTTCATCAGCGGGATACAGTACCTAGCAGGCTTGTCCACTCTGCCT
LysHisMetTrpAsnPheIleSerGlyIleGlnTyrLeuAlaGlyLeuSerThrLeuPro
               65                  70                  75                  80
```

```
SEQ ID NO : 3
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human hepatitis C virus gene


                 10        20        30        40        50        60
    CAAGAGTTCGACGAAATGGAAGAGTGCGCCTCACACCTTCCTTACATCGAACAGGGAATG
    GlnGluPheAspGluMetGluGluCysAlaSerHisLeuProTyrIleGluGlnGlyMet
                    5                  10                  15                  20

                 70        80        90       100       110       120
    CAGCTCGCCGAGCAATTCAAGCAGAAAGCGCTCGGTTTGCTGCAGACAGCTACCAAGCAA
    GlnLeuAlaGluGlnPheLysGlnLysAlaLeuGlyLeuLeuGlnThrAlaThrLysGln
                   25                  30                  35                  40

                130       140       150       160       170       180
    GCGGAGGCTGCAGCCCCCGTGGTGGAGTCCAAGTGGCGGGCCCTTGAGACTTTCTGGGCG
    AlaGluAlaAlaAlaProValValGluSerLysTrpArgAlaLeuGluThrPheTrpAla
                   45                  50                  55                  60

                190       200       210       220       230       240
    AAGCACATGTGGAATTTCATCAGCGGGATACAATACTTAGCAGGCTTATCCACTTTGCCT
    LysHisMetTrpAsnPheIleSerGlyIleGlnTyrLeuAlaGlyLeuSerThrLeuPro
                   65                  70                  75                  80
```

SEQ ID NO : 4
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
        10        20        30        40        50        60
CCGGCGTTCGATGAAATGGAAGAGTGTGCCTCACACCTCCCTTACATCGAACAAGGAATG
ProAlaPheAspGluMetGluGluCysAlaSerHisLeuProTyrIleGluGlnGlyMet
              5                  10                  15                  20

        70        80        90       100       110       120
CGGCTCGCCGAACAATTCAAGCAGAAGGCGCTCGGGTTGCTGCAAACGGCCACCAAGCAA
GlnLeuAlaGluGlnPheLysGlnLysAlaLeuGlyLeuLeuGlnThrAlaThrLysGln
             25                  30                  35                  40

       130       140       150       160       170       180
GCGGAGGCTGCCGCTCCCGTGGTGGAGTCCAAGTGGCGTACCCTTGAGGCCTTCTGGGCG
AlaGluAlaAlaAlaProValValGluSerLysTrpArgThrLeuGluAlaPheTrpAla
             45                  50                  55                  60

       190       200       210       220       230       240
AAGCACATGTGGAATTTCATCAGCGGGATACAATACCTAGCAGGCTTGTCCACTCTGCCT
LysHisMetTrpAsnPheIleSerGlyIleGlnTyrLeuAlaGlyLeuSerThrLeuPro
             65                  70                  75                  80
```

SEQ ID NO : 5
SEQUENCE TYPE : Nucleotide with corresponding protein
SEQUENCE LENGTH : 240 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
         10         20         30         40         50         60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
              5                  10                  15                  20

         70         80         90        100        110        120
CAGCTCGCCGAGCAATTCAAGCAGAAGGCGCTCGGGCTGCTGCAAACGGCCTCCAAGCAA
GlnLeuAlaGluGlnPheLysGlnLysAlaLeuGlyLeuLeuGlnThrAlaSerLysGln
             25                  30                  35                  40

        130        140        150        160        170        180
GCGGAGGCTGCTGCTCCCGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
             45                  50                  55                  60

        190        200        210        220        230        240
AAGCACATGTGGAATCTCATCAGCGGGATACAGTATCTAGCAGGCTTGTCCACCCTGCCT
LysHisMetTrpAsnLeuIleSerGlyIleGlnTyrLeuAlaGlyLeuSerThrLeuPro
             65                  70                  75                  80
```

SEQ ID NO : 6
SEQUENCE TYPE : Nucleotide with corresponding protein
SEQUENCE LENGTH : 209 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
        10        20        30        40        50        60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
             5                  10                  15                  20

        70        80        90       100       110       120
CAGCTCGCCGAGCAATTCAAGCAGAAGGCGCTCGGGCTGCTGCAAACAACCTCCAAGCAA
GlnLeuAlaGluGlnPheLysGlnLysAlaLeuGlyLeuLeuGlnThrThrSerLysGln
            25                  30                  35                  40

       130       140       150       160       170       180
GCGGAGGCTGCTGCTCCTGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
            45                  50                  55                  60

       190       200       210
AAGCACATGTGGAATCTCATCAGCGGGAT
LysHisMetTrpAsnLeuIleSerGly
            65                  70
```

SEQ ID NO : 7
SEQUENCE TYPE : Nucleotide with corresponding protein
SEQUENCE LENGTH : 209 base pairs

STRANDNESS : single
TOPOLOGY : linear
MOLECULE TYPE : genomic DNA

ORIGINAL SOURCE
ORGANISM : human

PROPERTIES : human hepatitis C virus gene

```
         10        20        30        40        50        60
CAGGAGTTTGATGAGATGGAGGAGTGCGCCTCACACCTTCCTTACATCGAACAAGGAATG
GlnGluPheAspGluMetGluGluCysAlaSerHisLeuProTyrIleGluGlnGlyMet
              5                  10                  15                  20

         70        80        90       100       110       120
CAGCTCGCCGAGCAATTCAAGCAGAAGGCGCTCGGGCTGCTGCAGACAACCTCCAAGCAA
GlnLeuAlaGluGlnPheLysGlnLysAlaLeuGlyLeuLeuGlnThrThrSerLysGln
             25                  30                  35                  40

        130       140       150       160       170       180
GCGGAGGCTGCTGCTCCCGTGGTAGAGTCCAAGTGGCAAGCCCTTGAGGCCTTCTGGGCG
AlaGluAlaAlaAlaProValValGluSerLysTrpGlnAlaLeuGluAlaPheTrpAla
             45                  50                  55                  60

        190       200       210
AAGCACATGTGGAATCTCATCAGCGGGAT
LysHisMetTrpAsnLeuIleSerGly
             65                  70
```

## Claims

1. A single-stranded DNA encoding a part of non-A, non-B hepatitis virus (NANBH) gene and comprising about 210 to 240 deoxyribonucleotides, a double-stranded DNA consisting of said single-stranded DNA and its complementary DNA, or a fragment thereof.

2. A single-stranded DNA having a nucleotide sequence selected from the group consisting of

10 20 30 40 50 60

A CAGGAGTTTGATGAGATGGAGGAGTGCGCCTCACACCTTCCTTACATCGAACAAGGAATG
B CGGGAGTTCGATGAGATGGAGGAGTGCGCTTCACACCTCCCTTACATCGAACAGGGAATG
C CAAGAGTTCGACGAAATGGAAGAGTGCGCCTCACACCTTCCTTACATCGAACAGGGAATG
D CCGGCGTTCGATGAAATGGAAGAGTGTGCCTCACACCTCCCTTACATCGAACAAGGAATG
E CAGGAGTTTGATGAGATGGAGGAGTGCGCCTCACACCTTCCTTACATCGAACAAGGAATG
F CAGGAGTTTGATGAGATGGAGGAGTGCGCCTCACACCTTCCTTACATCGAACAAGGAATG
G CAGGAGTTTGATGAGATGGAGGAGTGCGCCTCACACCTTCCTTACATCGAACAAGGAATG

70 80 90 100 110 120

A CAGCTCGCCGAGCAATTCAAGCAGAAGGCGCTCGGGCTGCTGCAAACAGCCTCCAAGCAA
B CAGCTCGCCGAGCAATTCAAGCAGAAGGCGCTCGGGCTGTTGCAGACAGCCACCAAGCAA
C CAGCTCGCCGAGCAATTCAAGCAGAAAGCGCTCGGTTTGCTGCAGACAGCTACCAAGCAA
D CGGCTCGCCGAACAATTCAAGCAGAAGGCGCTCGGGTTGCTGCAAACGGCCACCAAGCAA
E CAGCTCGCCGAGCAATTCAAGCAGAAGGCGCTCGGGCTGCTGCAAACGGCCTCCAAGCAA
F CAGCTCGCCGAGCAATTCAAGCAGAAGGCGCTCGGGCTGCTGCAAACAACCTCCAAGCAA
G CAGCTCGCCGAGCAATTCAAGCAGAAGGCGCTCGGGCTGCTGCAGACAACCTCCAAGCAA

130 140 150 160 170 180

A GCGGAGGCTGCTGCTCCCGTGGTAGAGTCCAAGTGGCAAGCCCTTGAGGCCTTCTGGGCG
B GCGGAGGCTGCAGCTCCCGTGGTCGAGTCTAAATGGCGGGCTCTTGAGACCTTCTGGGCG
C GCGGAGGCTGCAGCCCCCGTGGTGGAGTCCAAGTGGCGGGCCCTTGAGACTTTCTGGGCG
D GCGGAGGCTGCCGCTCCCGTGGTGGAGTCCAAGTGGCGTACCCTTGAGGCCTTCTGGGCG
E GCGGAGGCTGCTGCTCCCGTGGTAGAGTCCAAGTGGCAAGCCCTTGAGGCCTTCTGGGCG
F GCGGAGGCTGCTGCTCCTGTGGTAGAGTCCAAGTGGCAAGCCCTTGAGGCCTTCTGGGCG
G GCGGAGGCTGCTGCTCCCGTGGTAGAGTCCAAGTGGCAAGCCCTTGAGGCCTTCTGGGCG

190 200 210 220 230 240

A AAGCACATGTGGAACTTCATCAGCGGGATACAGTATCTAGCAGGCTTGTCCACCCTGCCT
B AAGCACATGTGGAATTTCATCAGCGGGATACAGTACCTAGCAGGCTTGTCCACTCTGCCT
C AAGCACATGTGGAATTTCATCAGCGGGATACAATACTTAGCAGGCTTATCCACTTTGCCT
D AAGCACATGTGGAATTTCATCAGCGGGATACAATACCTAGCAGGCTTGTCCACTCTGCCT
E AAGCACATGTGGAATCTCATCAGCGGGATACAGTATCTAGCAGGCTTGTCCACCCTGCCT
F AAGCACATGTGGAATCTCATCAGCGGGAT
G AAGCACATGTGGAATCTCATCAGCGGGAT

wherein each of symbols of A, C, G and T is a deoxyribonucleotide having adenine, cytosine, guanine or thymine base, a double-stranded DNA consisting of the single-stranded DNA and its complementary DNA, or a fragment thereof.

3. A single-stranded DNA having about 240 deoxyribonucleotides as claimed in Claim 1, which encodes an amino acid sequence of

```
                    5              10             15             20
X¹-X²-PheAspGluMetGluGluCysAlaSerHisLeuProTyrIleGluGlnGlyMet

                    25             30             35             40
GlnLeuAlaGluGlnPheLysGlnLysAlaLeuGlyLeuLeuGlnThrAlaX³-LysGln

                    45             50             55             60
AlaGluAlaAlaAlaProValValGluSerLysTrpX⁴-X⁵-LeuGluX⁶-PheTrpAla

                    65             70             75             80
LysHisMetTrpAsnX⁷-IleSerGlyIleGlnTyrLeuAlaGlyLeuSerThrLeuPro
```

wherein $X^1$ is Gln, Arg or Pro, $X^2$ is Gln or Ala, $X^3$ is Ser or Thr, $X^4$ is Gln or Arg, $X^5$ is Ala or Thr, $X^6$ is Ala or Thr, and $X^7$ is Phe or Leu.

4. A single-stranded DNA having about 210 deoxyribonucleotides as claimed in Claim 1, which encodes an amino acid sequence of

```
                    5              10             15             20
GlnGluPheAspGluMetGluGluCysAlaSerHisLeuProTyrIleGluGlnGlyMet

                    25             30             35             40
GlnLeuAlaGluGlnPheLysGlnLysAlaLeuGlyLeuLeuGlnThrThrSerLysGln

                    45             50             55             60
AlaGluAlaAlaAlaProValValGluSerLysTrpGlnAlaLeuGluAlaPheTrpAla

                    65      69
LysHisMetTrpAsnLeuIleSerGly.
```

5. A diagnosis of NANBH, characterized in that a polymerase chain reaction is carried out with use of a double-stranded fragment consisting of a single-stranded RNA derived from a serum sample of an individual to be diagnosed and its complementary single-stranded DNA fragment as well as a set of primers which are parts of a single-stranded DNA fragment encoding a part of NANBH virus gene and having about 210 to 240 deoxyribonucleotides.

6. A diagnosis as claimed in Claim 3, wherein the polymerase chain reaction is carried out in two stages, by using in the first stage a set of the primers of

GAGTGCGCCTCACACCTTCCTTACATCGAACAAGGA

and

CTGGGCGAAGCACATGTGGAACTTCATCAGCGGGAT

and using in the second stage a set of the primers of

CTTCCTTACATCGAACAAGGA

and

CTGGGCGAAGCACATGTGGAA.

7. A diagnosis as claimed in Claim 3, wherein the polymerase chain reaction is carried out in two stages, by using in the first stage a set of the primers of

CAGGAGTTTGATGAGATGGAGGAGTGCGCCTCACAC

and

GCTGCTGCTCCCGTGGTAGAGTCCAAGTGGCAAGCC

and using in the second stage a set of the primers of

ATGGAGGAGTGCGCCTCACAC

and

GTAGAGTCCAAGTGGCAAGCC.

8. A diagnosis as claimed in Claim 3, wherein the polymerase chain reaction is carried out in two stages, by using in the first stage a set of the primers of

GAGTGCGCCTCACACCTTCCTTACATCGAACAAGGA

and

CTGGGCGAAGCACATGTGGAATCTCATCAGCGGGAT

and using in the second stage a set of the primers of

CTTCCTTACATCGAACAAGGA

and

CTGGGCGAAGCACATGTGGAA.

9. A diagnosis as claimed in Claim 3, wherein the polymerase chain reaction is carried out in two stages, by using in the first stage a set of the primers of

CAGGAGTTTGATGAGATGGAGGAGTGCGCCTCACAC

and

GCTGCTGCTCCTGTGGTAGAGTCCAAGTGGCAAGCC

and using in the second stage a set of the primers of

ATGGAGGAGTGCGCCTCACAC

and

GTAGAGTCCAAGTGGCAAGCC.

# FIG. 1

```
          10        20        30        40        50        60
A   CAGGAGTTTGATGAGATGGAGGAGTGCGCCTCACACCTTCCTTACATCGAACAAGGAATG
B   CGGGAGTTCGATGAGATGGAGGAGTGCGCTTCACACCTCCCTTACATCGAACAGGGAATG
C   CAAGAGTTCGACGAAATGGAAGAGTGCGCCTCACACCTTCCTTACATCGAACAGGGAATG
D   CCGGCGTTCGATGAAATGGAAGAGTGTGCCTCACACCTCCCTTACATCGAACAAGGAATG
E   CAGGAGTTTGATGAGATGGAGGAGTGCGCCTCACACCTTCCTTACATCGAACAAGGAATG
F   CAGGAGTTTGATGAGATGGAGGAGTGCGCCTCACACCTTCCTTACATCGAACAAGGAATG
G   CAGGAGTTTGATGAGATGGAGGAGTGCGCCTCACACCTTCCTTACATCGAACAAGGAATG

          70        80        90       100       110       120
A   CAGCTCGCCGAGCAATTCAAGCAGAAGGCGCTCGGGCTGCTGCAAACAGCCTCCAAGCAA
B   CAGCTCGCCGAGCAATTCAAGCAGAAGGCGCTCGGGCTGTTGCAGACAGCCACCAAGCAA
C   CAGCTCGCCGAGCAATTCAAGCAGAAAGCGCTCGGTTTGCTGCAGACAGCTACCAAGCAA
D   CGGCTCGCCGAACAATTCAAGCAGAAGGCGCTCGGGTTGCTGCAAACGGCCACCAAGCAA
E   CAGCTCGCCGAGCAATTCAAGCAGAAGGCGCTCGGGCTGCTGCAAACGGCCTCCAAGCAA
F   CAGCTCGCCGAGCAATTCAAGCAGAAGGCGCTCGGGCTGCTGCAAACAACCTCCAAGCAA
G   CAGCTCGCCGAGCAATTCAAGCAGAAGGCGCTCGGGCTGCTGCAGACAACCTCCAAGCAA

         130       140       150       160       170       180
A   GCGGAGGCTGCTGCTCCCGTGGTAGAGTCCAAGTGGCAAGCCCTTGAGGCCTTCTGGGCG
B   GCGGAGGCTGCAGCTCCCGTGGTCGAGTCTAAATGGCGGGCTCTTGAGACCTTCTGGGCG
C   GCGGAGGCTGCAGCCCCCGTGGTGGAGTCCAAGTGGCGGGCCCTTGAGACTTTCTGGGCG
D   GCGGAGGCTGCCGCTCCCGTGGTGGAGTCCAAGTGGCGTACCCTTGAGGCCTTCTGGGCG
E   GCGGAGGCTGCTGCTCCCGTGGTAGAGTCCAAGTGGCAAGCCCTTGAGGCCTTCTGGGCG
F   GCGGAGGCTGCTGCTCCTGTGGTAGAGTCCAAGTGGCAAGCCCTTGAGGCCTTCTGGGCG
G   GCGGAGGCTGCTGCTCCCGTGGTAGAGTCCAAGTGGCAAGCCCTTGAGGCCTTCTGGGCG

         190       200       210       220       230       240
A   AAGCACATGTGGAACTTCATCAGCGGGATACAGTATCTAGCAGGCTTGTCCACCCTGCCT
B   AAGCACATGTGGAATTTCATCAGCGGGATACAGTACCTAGCAGGCTTGTCCACTCTGCCT
C   AAGCACATGTGGAATTTCATCAGCGGGATACAATACTTAGCAGGCTTATCCACTTTGCCT
D   AAGCACATGTGGAATTTCATCAGCGGGATACAATACCTAGCAGGCTTGTCCACTCTGCCT
E   AAGCACATGTGGAATCTCATCAGCGGGATACAGTATCTAGCAGGCTTGTCCACCCTGCCT
F   AAGCACATGTGGAATCTCATCAGCGGGAT
G   AAGCACATGTGGAATCTCATCAGCGGGAT
```

# FIG. 2

| | 5 10 15 20 |
|---|---|
| A | GlnGluPheAspGluMetGluGluCysAlaSerHisLeuProTyrIleGluGlnGlyMet |
| B | ArgGluPheAspGluMetGluGluCysAlaSerHisLeuProTyrIleGluGlnGlyMet |
| C | GlnGluPheAspGluMetGluGluCysAlaSerHisLeuProTyrIleGluGlnGlyMet |
| D | ProAlaPheAspGluMetGluGluCysAlaSerHisLeuProTyrIleGluGlnGlyMet |
| E | GlnGluPheAspGluMetGluGluCysAlaSerHisLeuProTyrIleGluGlnGlyMet |
| F | GlnGluPheAspGluMetGluGluCysAlaSerHisLeuProTyrIleGluGlnGlyMet |
| G | GlnGluPheAspGluMetGluGluCysAlaSerHisLeuProTyrIleGluGlnGlyMet |

| | 25 30 35 40 |
|---|---|
| A | GlnLeuAlaGluGlnPheLysGlnLysAlaLeuGlyLeuLeuGlnThrAlaSerLysGln |
| B | GlnLeuAlaGluGlnPheLysGlnLysAlaLeuGlyLeuLeuGlnThrAlaThrLysGln |
| C | GlnLeuAlaGluGlnPheLysGlnLysAlaLeuGlyLeuLeuGlnThrAlaThrLysGln |
| D | GlnLeuAlaGluGlnPheLysGlnLysAlaLeuGlyLeuLeuGlnThrAlaThrLysGln |
| E | GlnLeuAlaGluGlnPheLysGlnLysAlaLeuGlyLeuLeuGlnThrAlaSerLysGln |
| F | GlnLeuAlaGluGlnPheLysGlnLysAlaLeuGlyLeuLeuGlnThrThrSerLysGln |
| G | GlnLeuAlaGluGlnPheLysGlnLysAlaLeuGlyLeuLeuGlnThrThrSerLysGln |

| | 45 50 55 60 |
|---|---|
| A | AlaGluAlaAlaAlaProValValGluSerLysTrpGlnAlaLeuGluAlaPheTrpAla |
| B | AlaGluAlaAlaAlaProValValGluSerLysTrpArgAlaLeuGluThrPheTrpAla |
| C | AlaGluAlaAlaAlaProValValGluSerLysTrpArgAlaLeuGluThrPheTrpAla |
| D | AlaGluAlaAlaAlaProValValGluSerLysTrpArgThrLeuGluAlaPheTrpAla |
| E | AlaGluAlaAlaAlaProValValGluSerLysTrpGlnAlaLeuGluAlaPheTrpAla |
| F | AlaGluAlaAlaAlaProValValGluSerLysTrpGlnAlaLeuGluAlaPheTrpAla |
| G | AlaGluAlaAlaAlaProValValGluSerLysTrpGlnAlaLeuGluAlaPheTrpAla |

| | 65 70 75 80 |
|---|---|
| A | LysHisMetTrpAsnPheIleSerGlyIleGlnTyrLeuAlaGlyLeuSerThrLeuPro |
| B | LysHisMetTrpAsnPheIleSerGlyIleGlnTyrLeuAlaGlyLeuSerThrLeuPro |
| C | LysHisMetTrpAsnPheIleSerGlyIleGlnTyrLeuAlaGlyLeuSerThrLeuPro |
| D | LysHisMetTrpAsnPheIleSerGlyIleGlnTyrLeuAlaGlyLeuSerThrLeuPro |
| E | LysHisMetTrpAsnLeuIleSerGlyIleGlnTyrLeuAlaGlyLeuSerThrLeuPro |
| F | LysHisMetTrpAsnLeuIleSerGly |
| G | LysHisMetTrpAsnLeuIleSerGly |

FIG. 3

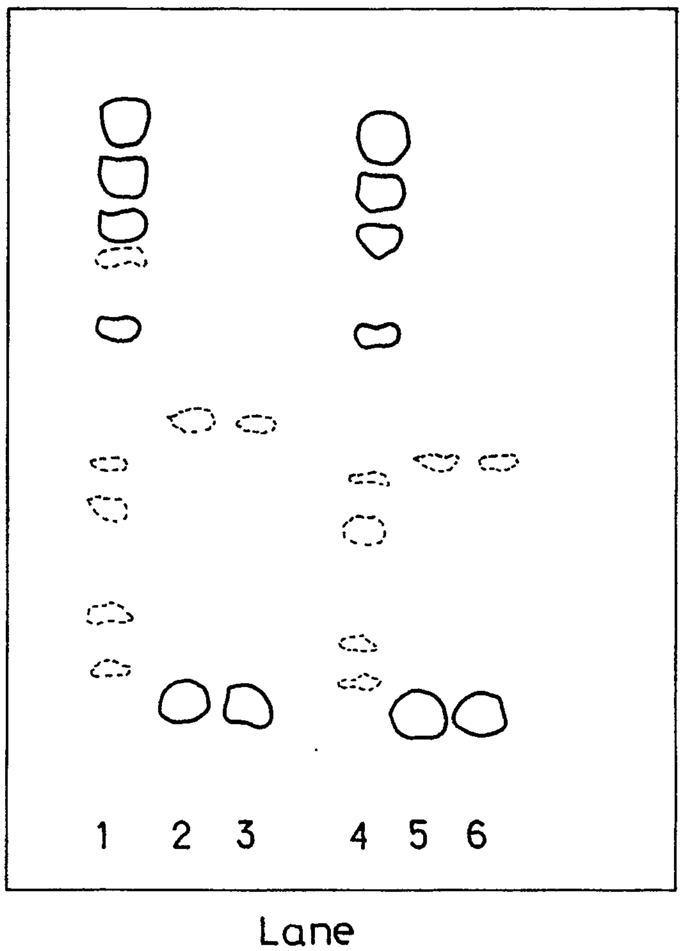

FIG. 4

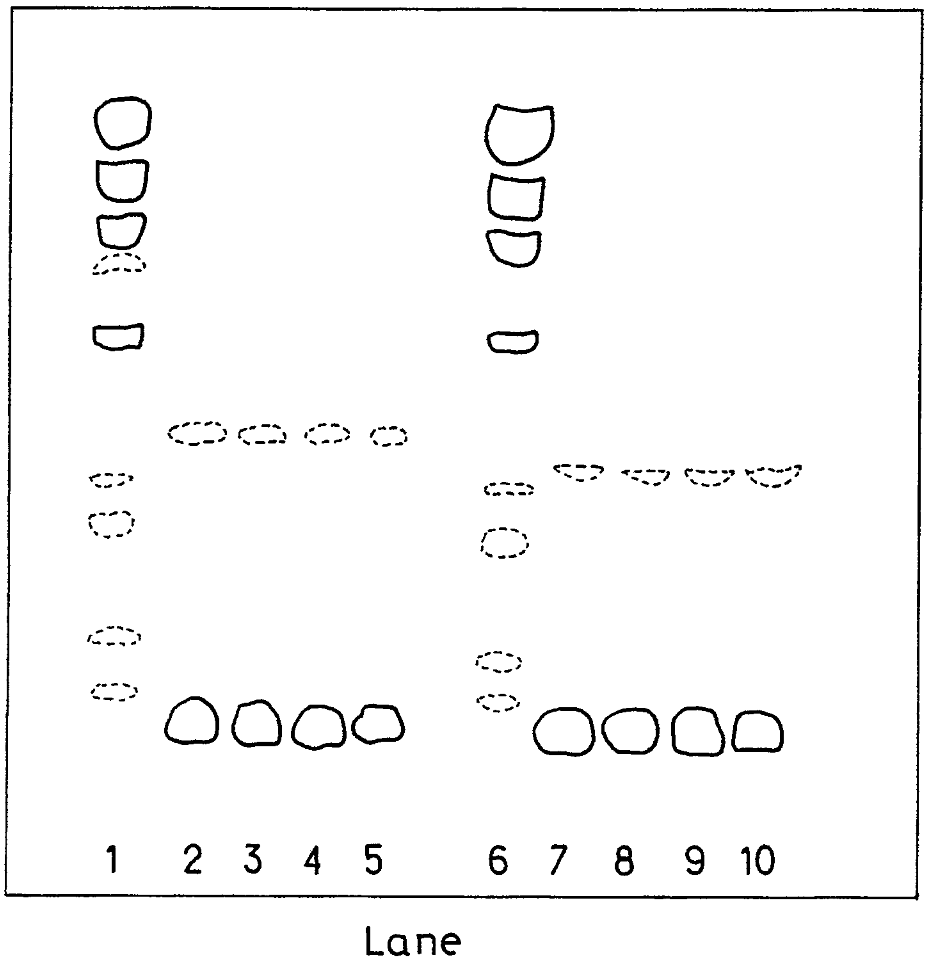

European Patent Office

# EUROPEAN SEARCH REPORT

Application Number

**EP 90 12 5293**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 318 216 (CHIRON CORPORATION) * whole document; in particular figures 3,47-4,47-5 * | 1-9 | C 07 K 15/00<br>C 12 N 15/40<br>C 12 Q 1/70<br>A 61 K 39/29 |
| P,X | EP-A-0 388 232 (CHIRON CORPORATION) * whole document; in particular figures 16-5,16-6 * | 1-9 | |
| X | EP-A-0 279 460 (DR. R. SEELIG et al.) * whole document; in particular page 7, column 1, example 9 * | 1,5 | |
| X<br><br>Y | EP-A-0 293 274 (MITSUBISHI CHEMICAL INDUSTRIES LTD.) * whole document * | 1<br><br>5 | |
| Y | EP-A-0 229 701 (CETUS CORPORATION) * whole document * | 5 | |
| D,A | PATENT ABSTRACTS OF JAPAN vol. 12, no. 326 (C-525), 5 September 1988; & JP - A - 63091328 (MITSUBISHI CHEM. IND. LTD.) 22.04.1988 * abstract * | 1 | |

| TECHNICAL FIELDS SEARCHED (Int. Cl.5) |
|---|
| C 07 K<br>15/00<br>C 07 K 15/12<br>C 12 N 15/33<br>C 12 N 15/36<br>C<br>12 N 15/40 |

The present search report has been drawn up for all claims

| Place of search | Date of completion of search | Examiner |
|---|---|---|
| Berlin | 15 February 91 | JULIA P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document